# EUROPÄISCHE PATENTANMELDUNG

(11) **EP 1 574 496 A1**
(43) Veröffentlichungstag der Anmeldung: **14.09.2005**
(21) Anmeldenummer: 04005507.1
(22) Anmeldetag: 08.03.2004
(51) Int. Cl.: C07C 67/317

(54) **Herstellung von Verbindungen mit der CHF2- oder CHF-Gruppe**

(71) Anmelder: Solvay Fluor GmbH, 30173 Hannover (DE)
(72) Erfinder: Braun, Max, 30900 Wedemark (DE)
(74) Vertreter: Fischer, Reiner

(57) **Zusammenfassung**

Verbindungen mit einer CHF₂- oder CHF-Gruppe können aus den entsprechenden Verbindungen mit einer CCIF₂- oder CCIF-Gruppe oder der entsprechenden Brom- oder Jodverbindung und Zink in Anwesenheit eines Alkohols hergestellt werden. Bevorzugt werden Monoester mit einer CHF₂-Gruppe oder Diester mit einer CHF-Gruppe auf diese Weise hergestellt.

## Beschreibung

Die Erfindung bezieht sich auf ein Verfahren zur Herstellung von Verbindungen mit einer CHF₂- oder CHF-Gruppe durch Hydrodehalogenierung, vorzugsweise Hydrodechlorierung aus entsprechenden Brom-, Jod- oder Chlorverbindungen, bevorzugt aus Verbindungen mit einer CCIF₂- oder CCIF-Gruppe.

Verbindungen mit einer CHF₂- oder CHF-Gruppe, insbesondere Esterverbindungen und Diesterverbindungen, sind wertvolle Zwischenprodukte in der chemischen Synthese.

Es ist bereits bekannt, solche Verbindungen aus entsprechenden ChlorVerbindungen und Austausch des Chloratoms gegen Wasserstoff herzustellen. Takashi Tsukamoto und Tomoya Kitazume geben in J. Chem. Soc. Perkin Trans. 1993, Seiten 1177 bis 1181 an, dass Chlordifluoressigsäureethylester mit Zink in Dimethylformamid und anschließender saurer Hydrolyse quantitativ Difluoressigsäureethylester ergibt (siehe Seite 1177, linke Spalte). Es ist jedoch nicht angegeben, wie der Ester isoliert werden könnte.

Aufgabe der vorliegenden Erfindung ist es, ein verbessertes Verfahren zur Herstellung von Verbindungen mit einer CHF2- oder CHF-Gruppe, besonders von entsprechenden Ester- oder Diesterverbindungen, anzugeben. Diese Aufgabe wird durch das erfindungsgemäße Verfahren gelöst.

Das erfindungsgemäße Verfahren zur Herstellung von Verbindungen mit einer CFₙH-Gruppe aus einer CFₙX-Gruppe, wobei n für 1 oder 2 steht und X Brom, Jod oder vorzugsweise Chlor bedeutet, und Zink wird in Anwesenheit eines Alkohols durchgeführt. Der Alkohol dient als Protonenquelle für die Reaktion. Dimethylformamid oder andere Carbonsäureamide sind bevorzugt nicht im Reaktionsgemisch enthalten. Bevorzugt sind auch keine anderen Lösungsmittel während der Umsetzung anwesend.

Eine bevorzugte Ausführungsform des erfindungsgemäßen Verfahrens ist dadurch gekennzeichnet, dass man Verbindungen mit einer oder zwei CFₙHC(O)-Gruppen aus einer Verbindung mit einer oder zwei CFₙXC(O)-Gruppe herstellt, wobei n und X die vorgenannte Bedeutung besitzen. Bevorzugt stellt man Verbindungen mit einer oder zwei CFₙHC(O)-Gruppe aus einer Verbindung mit einer oder zwei CFₙClC(O)-Gruppe her.

Besonders bevorzugt stellt man einen Ester der Formel R¹ CFHC(O)OR² her, worin R¹ steht für F; für C1-C5-Alkyl; oder für C1-C5-Alkyl, das durch mindestens 1 Fluoratom substituiert ist; und und R² steht für C1-C5-Alkyl; oder für C1-C5-Alkyl, das durch mindestens 1 Fluoratom substituiert ist. Weiterhin bevorzugt stellt man einen Diester der Formel R³OC(O)CFHC(O)OR³ her, worin R³ steht für C1-C5-Alkyl; oder für C1-C5-Alkyl, das durch mindestens 1 Fluoratom substituiert ist.

R¹ steht ganz besonders bevorzugt für F oder C1-C3, welches teilfluoriert oder perfluoriert ist.

R² und R³ stehen bevorzugt für Methyl, Ethyl, n-Propyl oder i-Propyl.

R¹ steht bevorzugt für F oder CF₃.

Der als Protonenquelle verwendete Alkohol entspricht zweckmäßigerweise dem Rest R² bzw. R³.

Gemäß einer Ausführungsform stellt man den Ester in situ aus dem entsprechenden Säurechlorid und Alkohol her. In diesem Fall ist der Alkohol nicht nur Protonenquelle, sondern dient auch zur Veresterung des Säurechlorids. Entsprechend mehr Alkohol muss eingesetzt werden. Da der Alkohol aber zweckmäßig im Überschuss als Lösungsmittel eingesetzt wird, stellt dies kein Problem dar.

Es kann vorteilhaft sein, die Umsetzung in Anwesenheit eines nichtprotischen Lösungsmittels durchzuführen. In diesem Fall verwendet man als nichtprotisches Lösungsmittel vorzugsweise mindestens überwiegend das herzustellende Produkt, z.B. den Ester mit einer CHF2- oder CHF-Gruppe. Nitrile sind vorzugsweise nicht als Lösungsmittel enthalten.

Pro auszutauschendem Chlor setzt man bevorzugt 0,9 bis 2,1 Equivalente Zink ein. Bevorzugt setzt man pro Chloratom etwa 1,1 bis 2 Zinkatome ein; ein stöchiometrischer Überschuß an Zink hat sich als vorteilhaft erwiesen.

Die Temperatur, bei der die Umsetzung zwischen brom-, jod- bzw. chlorhaltiger Ausgangsverbindung, Zink und Alkohol durchgeführt wird, liegt vorteilhaft zwischen 50 °C und dem Siedepunkt des entsprechenden Alkohols.

Die Isolation kann gemäß üblichen Methoden erfolgen.

Das erfindungsgemäße Verfahren hat den Vorteil, dass hohe Ausbeuten und hohe Selektivitäten erzielt werden. Lösungsmittel wie DMF sind auch schwerer zu entsorgen.

Die folgenden Beispiele sollen die Erfindung weiter erläutern, ohne sie in ihrem Umfang einzuschränken.

### Beispiele

### Beispiel 1: Herstellung von Difluoressigsäuremethylester aus Chlordifluoressigsäuremethylester und Zink

Allgemeine Durchführung: Ausgangsverbindung, Zink und Alkohol wurden in den angegebenen Proportionen und während der angegebenen Umsetzungsdauer und Temperatur miteinander umgesetzt. Das Abdestillieren des Methylesters ergab immer eine Fraktion eines Azeotrops von Difluoressigsäuremethylester und Methanol mit einem konstanten Siedepunkt von 64°C bei Umgebungsdruck. Diese Mischung wurde mit Benzotrifluorid als internem Standard versetzt und die Ausbeute durch ¹H- und ¹⁹F-NMR-Spektren bestätigt. Das Azeotrop kann zu anderen Difluoressigsäureverbindungen weiter umgesetzt werden. Mit NH₃ kann der Ester beispielsweise in quantitativer Ausbeute in das Difluoressigsäureamid umgewandelt werden.

Reaktionsbedingungen sind in Tabelle 1 zusammengestellt.

### Abkürzungen:

- CDFAMe: = Chlordifluoressigsäuremethylestre
- CDFAEt: = Chlordifluorethylester
- CDFACl: = Chlordifluoracetylchlorid
- MeOH: = Methanol
- EtOH: = Ethanol
- Eq.: = Equivalent
- Zn: = Zink

**Tabelle 1:**

| Reaktionsparameter und Analysendaten | | | | | | | | | | | |
|---|---|---|---|---|---|---|---|---|---|---|---|
| | CDFAMe + 1 Eq. Zn In MeOH, Reflux | | | CDFAMe + 1,5 Eq. Zn in MeOH, 50°C | | | | CDFAMe + 1,5 Eq. Zn in MeOH, Reflux | | | |
| | 1h | 2h | 3h | 1h | 2h | 3h | 4h | 1h | 2h | 3h | 4h |
| ClCF₂COOMe | 29,1¹⁾ | 17,1 | 14,3 | 72,5 | 36,8 | 12,2 | 4,9 | 43,7 | 10,9 | 1,1 | 0,3 |
| HCF₂COOMe | 58,4 | 68,8 | 70,3 | 26,8 | 59,2 | 81,1 | 84,8 | 55,9 | 88,3 | 95,6 | 95,8 |
| ClF₂COOH | 3,5 | 3,1 | 3,9 | 0,7 | 1,5 | 1,7 | 1,1 | 0,04 | 0,04 | 0 | 0 |
| HCF₂COOH | 9 | 11,1 | 11,6 | 0,3 | 2,6 | 5 | 9,1 | 0,4 | 0,8 | 3,3 | 4 |

| | | | | | | | | | | | |
|---|---|---|---|---|---|---|---|---|---|---|---|
| 1) Angaben in % | | | | | | | | | | | |

Es ist festzustellen, dass ein Überschuß Zink und Reflux-Temperatur des Methanols positiv für die Ausbeute ist.

### Beispiel 2: Herstellung von Difluoressigsäureethylester

Die Umsetzung wurde wie in Beispiel 1 durchgeführt, aber in siedendem Ethanol. in Tabelle 2 sind Reaktionsparameter und Analysendaten zusammengestellt.

**Tabelle 2:**

| Reaktionsparameter und Analysendate | | | |
|---|---|---|---|
| | CDFAEt + 1,5 Eq. Zn In EtOH, Reflux | | |
| | 1 h | 2h | 3h |
| ClCF₂COOEt | 0,8¹⁾ | 0 | 0 |
| HCF₂COOEt | 61,6 | 84,9 | 89,9 |
| ClF₂COOH | 18,1 | 3,3 | 0,4 |
| HCF₂COOH | 19,5 | 11,9 | 9,7 |

| | | | |
|---|---|---|---|
| 1) Angaben in % | | | |

### Beispiel 3: Herstellung von Difluoressigsäuremethylester aus in-situ hergestelltem Chlordifluoressigsäuremethylester

Durchführung: Zink wurde in Methanol vorgelegt und Chlordifluoracetylchlorid zugegeben. Die Reaktion verläuft exotherm unter Gasentwicklung. Verfahrensparameter und Analysendaten sind in Tabelle 3 zusammengestellt.

**Tabelle 3:**

| Reaktionsparameter und Analysendaten | | | |
|---|---|---|---|
| | CDFACl + 1,5 Eq. Zn In MeOH, 1 h, RT | CDFACI + 1,5 Eq. Zn In MeOH, 1 h, Reflux | CDFACI + 1,5 Eq. Zn In MeOH, 3 h, Reflux |
| ClCF₂COOMe | 52,1¹⁾ | 0 | 0 |
| HCF₂COOMe | 16,7 | 24,1 | 24,1 |
| ClF₂COOH | 28,6 | 35,4 | 35,4 |
| HCF₂COOH | 2,6 | 36,1 | 36,2 |

| | | | |
|---|---|---|---|
| 1) Angaben in % | | | |

Die Reaktion scheint nach 1 h Refluxieren beendet zu sein. Vermutlich reagiert das entstehende HCl sehr schnell mit dem Zink und bildet nascierenden Wasserstoff, der die ClCF₂-Gruppe aber nur zum Teil reduziert. Ein größerer Überschuß sollte das Ergebnis verbessern.

## Patentansprüche

1. Verfahren zur Herstellung von Verbindungen mit einer CFₙH-Gruppe aus einer CFₙX-Gruppe und Zink in Anwesenheit eines Alkohols, wobei n für 1 oder 2 steht und X Brom, Jod oder vorzugsweise Chlor bedeutet.

2. Verfahren nach Anspruch 1, **dadurch gekennzeichnet, dass** man Verbindungen mit einer CFₙHC(O)-Gruppe aus einer CFₙXC(O)-Gruppe, vorzugsweise einer CFₙClC(O)-Gruppe herstellt, wobei n und X die in Anspruch 1 genannte Bedeutung besitzen.

3. Verfahren nach Anspruch 1 oder 2, **dadurch gekennzeichnet, dass** man einen Ester der Formel R¹CFHC(O)OR² herstellt, worin R¹ steht für F; für C1-C5-Alkyl; oder für C1-C5-Alkyl, das durch mindestens 1 Fluoratom substituiert ist; und und R² steht für C1-C5-Alkyl; oder für C1-C5-Alkyl, das durch mindestens 1 Fluoratom substituiert ist; oder dass man einen Diester der Formel R³OC(O)CFHC(O)OR³ herstellt, worin R³ steht für C1-C5-Alkyl; oder für C1-C5-Alkyl, das durch mindestens 1 Fluoratom substituiert ist.

4. Verfahren nach Anspruch 3, **dadurch gekennzeichnet**, das R1 für F oder C1-C3 steht, welches teilfluoriert oder perfluoriert ist.

5. Verfahren nach Anspruch 3, **dadurch gekennzeichnet, dass** R² und R³ für Methyl, Ethyl, n-Propyl oder i-Propyl steht.

6. Verfahren nach Anspruch 3, **dadurch gekennzeichnet, dass** R¹ steht für F oder CF₃.

7. Verfahren nach Anspruch 3, **dadurch gekennzeichnet, dass** der Alkohol dem Rest R² oder R³ entspricht.

8. Verfahren nach Anspruch 3, **dadurch gekennzeichnet, dass** man den Ester in situ aus Säurechlorid und Alkohol herstellt.

9. Verfahren nach Anspruch 1, **dadurch gekennzeichnet, dass** man in Anwesenheit eines nichtprotischen Lösungsmittels arbeitet, vorzugsweise in Anwesenheit des Reaktionsprodukts als Lösungsmittel.

10. Das Azeotrop aus Difluoressigsäuremethylester und Methanol.
